# EUROPEAN PATENT APPLICATION

(11) **EP 3 725 245 A1**
(43) Date of publication of application: **21.10.2020**
(21) Application number: 20169358.7
(22) Date of filing: 14.04.2020
(51) Int. Cl.: A61B 17/34

(54) **BALLOON TROCAR INCLUDING A PIN**

(30) Priority: 15.04.2019 US 201962833826 P; 13.03.2020 US 202016818292
(71) Applicant: Covidien LP, Mansfield, MA 02048 (US)
(72) Inventor: GEORGE, Sebastian, 500050 Hyderabad (IN); BUYDA, Oksana, East Haven, CT 06513 (US)
(74) Representative: Maschio & Soames IP Ltd

(57) **Abstract**

A balloon trocar (100) includes a housing (200) and a cannula assembly (300). The housing (200) includes an inflation port (220). The housing (200) defines a cavity (210) configured to receive a seal therein. The cannula assembly (300) includes a tubular member (310) extending from the housing (200), an inflatable balloon (350) secured to the tubular member (310), and a pin (390). The tubular member (310) defines a lumen (302), a fluid channel (304), and an actuation channel (306). The lumen (302) is in communication with the cavity (210) of the housing (200). The lumen (302) is configured to receive a surgical instrument therethrough. The fluid channel (304) provides fluid communication between the inflatable balloon (350) and the inflation port (220). The actuation channel (306) is configured to slidably receive the pin (390) therein. The pin (390) includes a tip (390b) configured to pierce the inflatable balloon (350) to deflate the inflatable balloon (350).

## Description

### CROSS-REFERENCE TO RELATED APPLICATIONS

This application claims the benefit of and priority to U.S. Provisional Patent Application Serial No. 62/833,826 filed April 15, 2019, the entire disclosure of which is incorporated by reference herein.

### TECHNICAL FIELD

The present disclosure relates to a surgical access device. More particularly, the present disclosure relates to a balloon trocar having a pin configured to deflate a balloon.

### BACKGROUND OF RELATED ART

Minimally invasive surgery has become increasingly popular in recent years. Minimally invasive surgery eliminates the need to cut a large incision in a patient, thereby reducing discomfort, recovery time, and many of the deleterious side effects associated with traditional open surgery.

Trocar systems facilitate minimally invasive surgery by providing a working channel across an abdominal wall to facilitate the use of instruments within the abdominal cavity. Trocar systems typically include a cannula, which provides a working channel, and an obturator that is inserted into the working channel of the cannula and used to penetrate the body wall. To help ensure that the cannula remains fixed in position once placed, an inflatable balloon may be attached to a distal portion of the cannula.

### SUMMARY

In accordance with an embodiment of the disclosure, a balloon trocar includes a housing and a cannula assembly. The housing includes an inflation port. The housing defines a cavity configured to receive a seal therein. The cannula assembly includes a tubular member extending from the housing, an inflatable balloon secured to the tubular member, and a pin. The tubular member defines a lumen, a fluid channel, and an actuation channel. The lumen is in communication with the cavity of the housing. The lumen is configured to receive a surgical instrument therethrough. The fluid channel provides fluid communication between the inflatable balloon and the inflation port. The actuation channel is configured to slidably receive the pin therein. The pin includes a tip configured to pierce the inflatable balloon to deflate the inflatable balloon.

In an embodiment, the pin may include a proximal portion having a protrusion configured to be pushed distally to cause axial displacement of the pin.

In another embodiment, the tubular member of the cannula assembly may include a proximal portion defining a cutout configured to engage the proximal portion of the pin.

In yet another embodiment, the protrusion of the pin may be configured to extend outwardly through the cutout of the tubular member.

In still yet another embodiment, the proximal portion of the tubular member may include a stop configured to engage the protrusion of the pin to inhibit axial displacement of the protrusion beyond the stop.

In still yet another embodiment, the stop of the tubular member may be adjacent a distal portion of the cutout.

In an embodiment, the pin may be transitionable between a retracted position, in which, the tip of the pin is disposed within the actuation channel, and an extended position, in which, the tip of the pin extends out of the actuation channel to engage the inflatable balloon.

In another embodiment, the fluid channel and the actuation channel may diametrically oppose each other.

In yet another embodiment, the actuation channel of the tubular member may include a distal end portion having a ramp configured to direct the tip of the pin radially outward, toward the inflatable balloon.

In still yet another embodiment, the fluid port may be configured to be fluidly coupled to a fluid supply.

In an embodiment, the actuation channel may extend along a length of the tubular member.

In another embodiment, the pin may be formed of a resilient material.

In yet another embodiment, the cannula assembly may further include an anchor collar disposed on the tubular member. The anchor collar may be configured to form a seal against tissue interposed between the anchor collar and the inflatable balloon.

In an embodiment, the anchor collar may further include a clamp configured to selectively secure the anchor collar to a position along the tubular member.

In yet another embodiment, the anchor collar may be formed of a compressible material.

In yet another embodiment, the tip of the pin may be sharp.

In still yet another embodiment, the housing may further include an insufflation port.

### BRIEF DESCRIPTION OF THE DRAWINGS

Various embodiments of the present disclosure are illustrated herein with reference to the accompanying drawings, wherein:
FIG. 1 is a perspective view of a balloon trocar in accordance with an embodiment of the disclosure;
FIG. 2 is a side view of the balloon trocar of FIG. 1 with an anchor collar of a cannula assembly removed, illustrating a pin configured to deflate an inflatable balloon of the balloon trocar;
FIG. 3 is a partial side view of a tubular member of the balloon trocar of FIG. 1, illustrating a proximal end portion of the pin; and
FIG. 4 is a partial cross-sectional view of a distal end portion of the cannula assembly of the balloon trocar of FIG. 1.

### DETAILED DESCRIPTION

Embodiments of the presently disclosed surgical access device are described in detail with reference to the drawings, wherein like reference numerals designate corresponding elements in each of the several views. As used herein, the term "distal" refers to that portion of the instrument, or component thereof which is farther from the user while the term "proximal" refers to that portion of the instrument or component thereof which is closer to the user.

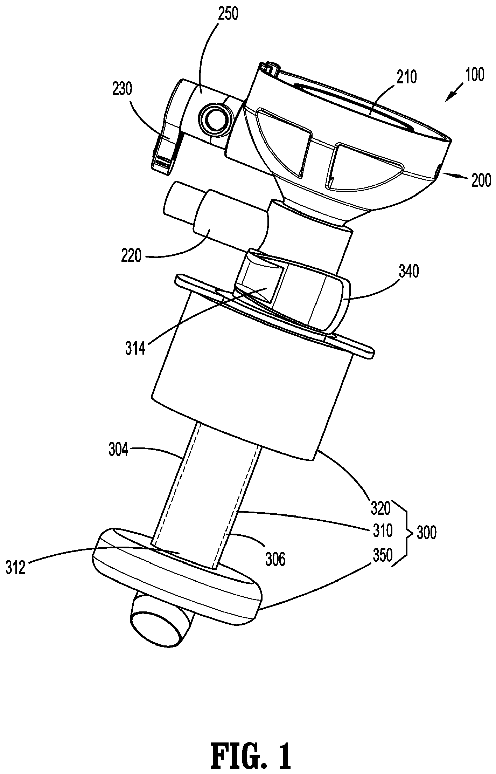

FIG. 1 illustrates a balloon trocar 100 in accordance with an embodiment of the disclosure. The balloon trocar 100 is configured to be placed through a body wall, such as an abdominal wall, and into a body cavity, such as an abdominal cavity, in a typical laparoscopic procedure. The body cavity is insufflated with fluid, to expand the body wall and provide a working space for the laparoscopic procedure. The balloon trocar 100 includes a housing 200 and a cannula assembly 300 extending distally from the housing 200. The components of the balloon trocar 100 may be formed of suitable biocompatible materials such as medical grade metals (e.g., stainless steel), polymeric materials (e.g., polycarbonate), or combinations thereof.

The housing 200 defines a cavity 210 to receive a surgical instrument (not shown) therethrough. A seal assembly (not shown) may be disposed within the cavity 210 in order to maintain positive pressure within the body cavity. If positive pressure is lost, the procedure may be compromised. The seal assembly may include, e.g., a duck bill or zero-closure seal positioned in the cavity 210. For example, the zero-closure seal may be formed of a suitable resilient material (e.g., silicone) and be configured to inhibit fluids from exiting proximally through the housing 200 in the absence of a surgical instrument (e.g., an endoscope) inserted therethrough. The housing further includes a fluid port 220 and an insufflation port 250. The insufflation port 250 may include a valve 230 having a lever 232 to adjust the flow rate of a fluid through the valve 230. An example of a suitable seal assembly usable with the balloon trocar 100 is described in U.S. Patent No. 10,022,149, issued on July 17, 2018, the entire contents of which are hereby incorporated by reference.

With continued reference to FIG. 1, the cannula assembly 300 includes a tubular member 310, an inflatable balloon 350 at a distal end portion 312 of the tubular member 310, and an anchor collar 320 selectively securable on the tubular member 310. In an embodiment, the tubular member 310 may be partially or completely transparent, translucent, or opaque. The inflatable balloon 350 is configured to fit snugly around the tubular member 310 in the uninflated condition. The inflatable balloon 350 may be bonded to the tubular member 310. For example, the inflatable balloon 350 may be attached to the tubular member 310 using known techniques such as RF welding, ultrasonic welding, adhesives, etc. The inflatable balloon 350 may be made to take on one of various shapes upon inflation thereof. For example, the inflatable balloon 350 may include a substantially toroid shape upon inflation. In another embodiment, the balloon may include a disc shape upon inflation. In another embodiment, the inflatable balloon 350 may be a fluted balloon.

The inflatable balloon 350 is inflated after the tubular member 310 is properly placed through the body wall and into the body cavity. An inflation fluid is supplied to the inflatable balloon 350 via the inflation port 220. The inflatable balloon 350 is configured to be held against an interior surface of the body wall by a counter-force that is associated with the anchor collar 320. The anchor collar 320 includes a clamp 340 configured to selectively secure the anchor collar 320 at any position along the length of the tubular member 310. In an embodiment, the tubular member 310 may be formed of, e.g., a polymeric material. In addition, the inflatable balloon 350 and the anchor collar 320 may be formed of compressible and/or resilient material. In this manner, the cannula assembly 300 forms a fluid-tight seal against adjacent tissue.

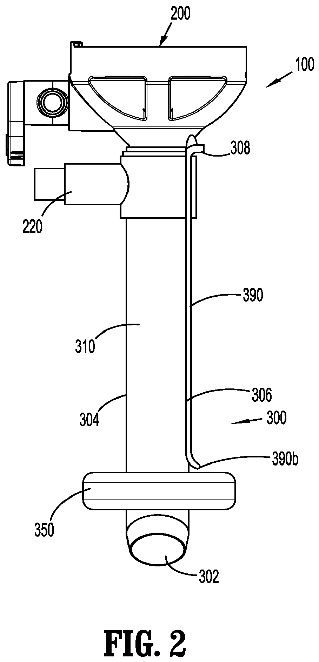

With reference to FIG. 2, the tubular member 310 of the cannula assembly 300 defines a lumen 302 in communication with the cavity 210 (FIG. 1) of the housing 200 to receive a surgical instrument (not shown) therethrough. In addition, the tubular member 310 further defines a fluid channel 304 in communication with the fluid port 220 and the inflatable balloon 350, and an actuation channel 306 configured to slidably receive a pin 390 therethrough. In an embodiment, it is contemplated that the fluid channel 304 and the actuation channel 306 may diametrically oppose each other such that use of the fluid channel 304 and the actuation channel 306 do not interfere with each other. The fluid channel 304 provides fluid communication between the fluid port 220 and the inflatable balloon 350 such that a fluid supplied through the fluid port 220 inflates the inflatable balloon 350. The pin 390 includes a distal tip 390b configured to pierce the inflatable balloon 350. The tip 390b may be sharp or, alternatively, blunt. The pin 390 further includes a protrusion portion 308 at a proximal end portion 390a of the pin 390. Under such a configuration, the pin 390 may be axially displaced to pierce the inflatable balloon 350. Specifically, the pin 390 is transitionable between a retracted position, in which, the tip 390b of the pin 390 is disposed within the actuation channel 306, and an extended position, in which, the tip 390b of the pin 390 extends out of the actuation channel 306 to engage the inflatable balloon 350.

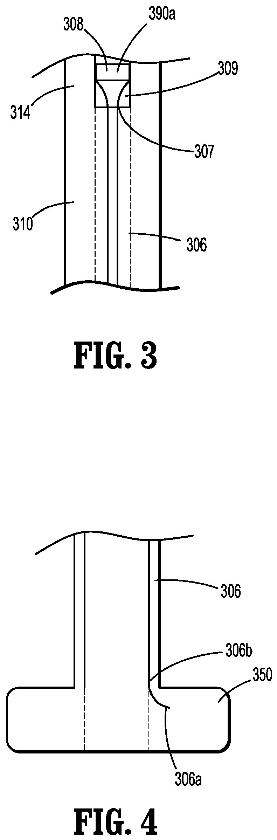

With reference to FIGS. 3 and 4, the proximal end portion 314 of the tubular member 310 of the cannula assembly 300 further defines a cutout 309 configured to receive the protrusion portion 308 at the proximal end portion 390a of the pin 390. The cutout 309 also includes a stop 307 configured to inhibit axial displacement of the protrusion portion 308 beyond the stop 307. In this manner, the amount of axial displacement of the pin 390 may be predetermined to inhibit any injury or trauma to tissue. Prior to actuation of the pin 390, the protrusion portion 308 may be frictionally secured to the cutout 309. However, a clinician may push the protrusion portion 308 distally to axially advance the pin 390 such that the distal tip 390b (FIG. 2) of the pin 390 extends out of the actuation channel 306 and pierces the inflatable balloon 350, thereby deflating the inflatable balloon 350. In this manner, the clinician may quickly deflate the inflatable balloon 350, rather than having to deflate the inflatable balloon 350 by other means such as, e.g., use of a syringe. In addition, by piercing the inflatable balloon 350 with the pin 390, the damaged inflatable balloon 350 renders the balloon trocar 100 inoperable, thereby inhibiting any reuse or reprocessing of a single-use balloon trocar 100 by a third party.

With particular reference now to FIG. 4, a distal end portion 306b of the actuation channel 306 may include a ramp 306a to direct the pin 390 radially outward, i.e., direct the distal tip 390b (FIG. 2) of the pin 390 towards the inflatable balloon 350.

In use, an incision is made in a body wall to gain entry to a body cavity, such as the abdominal cavity. The distal end portion 312 of the tubular member 310 of the balloon trocar 100 is inserted through the incision until the inflatable balloon 350 at the distal end portion 312 of the tubular member 310 is within the body cavity. At this time, a fluid supply such as e.g., a syringe (not shown), may be connected to the fluid port 220 of the housing 200 to supply an inflation fluid to the inflatable balloon 350. In order to seal the inflatable balloon 350 against the interior surface of the body wall, the anchor collar 320 may be slidably moved along the tubular member 310 while pulling the balloon trocar 100 proximally until the inflatable balloon 350 is pressed against the inner surface of the body wall and the anchor collar 320 is compressed against the outer surface of the body wall. At this time, the clamp 340 may be used to lock the position of the anchor collar 320 on the tubular member 310, thereby maintaining compression of the inflatable balloon 350 against the interior surface of the body wall and compression of the anchor collar 320 against the exterior surface of the body wall. With the incision sealed, the body cavity may be insufflated with CO₂, a similar gas, or another insufflation fluid. Surgical instruments (not shown) may be inserted through the lumen 302 of the balloon trocar 100 to perform desired surgical procedures. To deflate the inflatable balloon 350 for removal of the balloon trocar 100 from the body cavity, the clinician may press the protrusion portion 308 of the pin 390 until the protrusion portion 308 engages the stop 307 of the tubular member 310. At this time, the distal tip 390b pierces the inflatable balloon 350 and deflates the inflatable balloon 350. In this manner, the clinician may reduce the amount of time needed to deflate the inflatable balloon 350 and to remove the balloon trocar 100 from the body cavity. In addition, the use of the pin 390 damages the inflatable balloon 350 at the end of the procedure, which renders the balloon trocar 100 inoperable, thereby inhibiting any reuse or reprocessing of a single-use balloon trocar 100 by a third party

Persons skilled in the art will understand that the devices and methods specifically described herein and illustrated in the accompanying drawings are non-limiting exemplary embodiments. It is envisioned that the elements and features illustrated or described in connection with one exemplary embodiment may be combined with the elements and features of another without departing from the scope of the present disclosure. One skilled in the art will appreciate further features and advantages of the disclosure based on the above-described embodiments. Accordingly, the disclosure is not to be limited by what has been particularly shown and described, except as indicated by the appended claims.

The invention may be described by reference to the following numbered paragraphs:-
1. A balloon trocar comprising:
   a housing including an inflation port, the housing defining a cavity configured to receive a seal therein;
   a cannula assembly including a tubular member extending from the housing, an inflatable balloon secured to the tubular member, and a pin, the tubular member defining:
      a lumen in communication with the cavity of the housing, the lumen configured to receive a surgical instrument therethrough;
      a fluid channel providing fluid communication between the inflatable balloon and the inflation port; and
      an actuation channel configured to slidably receive the pin therein,
   wherein the pin includes a tip configured to pierce the inflatable balloon to deflate the inflatable balloon.
2. The balloon trocar according to paragraph 1, wherein the pin includes a proximal portion having a protrusion configured to be pushed distally to cause axial displacement of the pin.
3. The balloon trocar according to paragraph 2, wherein the tubular member of the cannula assembly includes a proximal portion defining a cutout configured to engage the proximal portion of the pin.
4. The balloon trocar according to paragraph 3, wherein the protrusion of the pin is configured to extend outwardly through the cutout of the tubular member.
5. The balloon trocar according to paragraph 3, wherein the proximal portion of the tubular member includes a stop configured to engage the protrusion of the pin to inhibit axial displacement of the protrusion beyond the stop.
6. The balloon trocar according to paragraph 5, wherein the stop of the tubular member is adjacent a distal portion of the cutout.
7. The balloon trocar according to paragraph 1, wherein the pin is transitionable between a retracted position, in which the tip of the pin is disposed within the actuation channel, and an extended position, in which the tip of the pin extends out of the actuation channel to engage the inflatable balloon.
8. The balloon trocar according to paragraph 1, wherein the fluid channel and the actuation channel diametrically oppose each other.
9. The balloon trocar according to paragraph 1, wherein the actuation channel of the tubular member includes a distal end portion having a ramp configured to direct the tip of the pin radially outward, toward the inflatable balloon.
10. The balloon trocar according to paragraph 1, wherein the fluid port is configured to be fluidly coupled to a fluid supply.
11. The balloon trocar according to paragraph 1, wherein the actuation channel extends along a length of the tubular member.
12. The balloon trocar according to paragraph 1, wherein the pin is formed of a resilient material.
13. The balloon trocar according to paragraph 1, wherein the cannula assembly further includes an anchor collar disposed on the tubular member, the anchor collar configured to form a seal against tissue interposed between the anchor collar and the inflatable balloon.
14. The balloon trocar according to paragraph 11, wherein the anchor collar further includes a clamp configured to selectively secure the anchor collar to a position along the tubular member.
15. The balloon trocar according to paragraph 11, wherein the anchor collar is formed of a compressible material.
16. The balloon trocar according to paragraph 1, wherein the tip of the pin is sharp.
17. The balloon trocar according to paragraph 1, wherein the housing further includes an insufflation port.

## Claims

1. A balloon trocar comprising:
a housing including an inflation port, the housing defining a cavity configured to receive a seal therein;
a cannula assembly including a tubular member extending from the housing, an inflatable balloon secured to the tubular member, and a pin, the tubular member defining:
a lumen in communication with the cavity of the housing, the lumen configured to receive a surgical instrument therethrough;
a fluid channel providing fluid communication between the inflatable balloon and the inflation port; and
an actuation channel configured to slidably receive the pin therein,
wherein the pin includes a tip configured to pierce the inflatable balloon to deflate the inflatable balloon.

2. The balloon trocar according to claim 1, wherein the pin includes a proximal portion having a protrusion configured to be pushed distally to cause axial displacement of the pin.

3. The balloon trocar according to claim 2, wherein the tubular member of the cannula assembly includes a proximal portion defining a cutout configured to engage the proximal portion of the pin.

4. The balloon trocar according to claim 3, wherein the protrusion of the pin is configured to extend outwardly through the cutout of the tubular member.

5. The balloon trocar according to claim 3, wherein the proximal portion of the tubular member includes a stop configured to engage the protrusion of the pin to inhibit axial displacement of the protrusion beyond the stop.

6. The balloon trocar according to claim 3 or claim 4, wherein the stop of the tubular member is adjacent a distal portion of the cutout.

7. The balloon trocar according to any preceding claim, wherein the pin is transitionable between a retracted position, in which the tip of the pin is disposed within the actuation channel, and an extended position, in which the tip of the pin extends out of the actuation channel to engage the inflatable balloon.

8. The balloon trocar according to any preceding claim, wherein the fluid channel and the actuation channel diametrically oppose each other.

9. The balloon trocar according to any preceding claim, wherein the actuation channel of the tubular member includes a distal end portion having a ramp configured to direct the tip of the pin radially outward, toward the inflatable balloon.

10. The balloon trocar according to any preceding claim, wherein the fluid port is configured to be fluidly coupled to a fluid supply.

11. The balloon trocar according to any preceding claim, wherein the actuation channel extends along a length of the tubular member.

12. The balloon trocar according to any preceding claim, wherein the pin is formed of a resilient material.

13. The balloon trocar according to claim 1, wherein the cannula assembly further includes an anchor collar disposed on the tubular member, the anchor collar configured to form a seal against tissue interposed between the anchor collar and the inflatable balloon;
preferably wherein the anchor collar further includes a clamp configured to selectively secure the anchor collar to a position along the tubular member; and/or wherein the anchor collar is formed of a compressible material.

14. The balloon trocar according to any preceding claim, wherein the tip of the pin is sharp.

15. The balloon trocar according to any preceding claim, wherein the housing further includes an insufflation port.
